# EUROPEAN PATENT SPECIFICATION

(11) **EP 2 968 221 B1**
(45) Date of publication and mention of the grant of the patent: **19.06.2019**
(21) Application number: 14719471.6
(22) Date of filing: 14.03.2014
(51) Int. Cl.: A61K 31/135, A61K 9/00, A61K 47/12, A61P 25/24

(54) **PHARMACEUTICAL COMPOSITION OF S-KETAMINE HYDROCHLORIDE**
PHARMAZEUTISCHE ZUSAMMENSETZUNG AUS S-KETAMIN-HYDROCHLORID
COMPOSITION PHARMACEUTIQUE D'HYDROCHLORURE DE KÉTAMINE S

(30) Priority: 15.03.2013 US 201361791505 P
(43) Date of publication of application: 20.01.2016
(60) Divisional application: 19151099.9
(73) Proprietor: Janssen Pharmaceutica NV, 2340 Beerse (BE)
(72) Inventor: BASSTANIE, Esther D. G., B-2240 Zandhoven (BE); BENTZ, Johanna, Newark, California 94560 (US); EMBRECHTS, Roger C.A., B-2360 Oud-Turnhout (BE); NIEMEIIJER, Nico Rudolph, NL-5685 JB Best (NL)
(74) Representative: Carpmaels & Ransford LLP
(86) International application number: PCT/US2014/027074
(87) International publication number: WO 2014/143646

(56) References cited:
- WO-A1-94/23711
- WO-A1-2014/031975
- WO-A2-2007/111880
- DE-A1-102007 009 888
- HUGE V ET AL: "Effects of low-dose intranasal (S)-ketamine in patients with neuropathic pain", EUROPEAN JOURNAL OF PAIN, SAUNDERS, LONDON, GB, vol. 14, no. 4, 1 April 2010 (2010-04-01), pages 387-394, XP026985780, ISSN: 1090-3801 [retrieved on 2009-09-03]
- JOAKIM JOHANSSON ET AL: "Prehospital analgesia using nasal administration of S-ketamine - a case se", SCANDINAVIAN JOURNAL OF TRAUMA, RESUSCITATION AND EMERGENCY MEDICINE, BIOMED CENTRAL LTD, LONDON UK, vol. 21, no. 1, 14 May 2013 (2013-05-14), page 38, XP021151671, ISSN: 1757-7241, DOI: 10.1186/1757-7241-21-38

## Description

### FIELD OF THE INVENTION

The present invention is directed to an aqueous formulation of S-ketamine hydrochloride, preferably for nasal administration, wherein the formulation does not contain an antimicrobial preservative.

### BACKGROUND OF THE INVENTION

Ketamine (a racemic mixture of the corresponding S- and R- enantiomers) is an NMDA receptor antagonist, with a wide range of effects in humans, including analgesia, anesthesia, hallucinations, dissociative effects, elevated blood pressure and bronchodilation. Ketamine is primarily used for the induction and maintenance of general anesthesia. Other uses include sedation in intensive care, analgesia (particularly in emergency medicine and treatment of bronchospasms. Ketamine has also been shown to be efficacious in the treatment of depression (particularly in those who have not responded to current anti-depressant treatment). In patients with major depressive disorders, ketamine has additionally been shown to produce a rapid antidepressant effect, acting within hours.

The *S*-ketamine enantiomer (or *S*-(+)-ketamine or esketamine) has higher potency or affinity for the NMDA receptor and thus potentially allowing for lower effective dosages; and is available for medical use, administered either IV (intravenously) or IM (intramuscularly), under the brand name KETANEST S.

In the formulation of a pharmaceutical compositing, the stability of the active ingredient is a primary concern. In general, drug substances are less stable in aqueous media than solid dosage forms, and it is important to properly stabilize and preserve liquid aqueous formulations such as solutions, suspensions, and emulsions. Acid-base reactions, acid or base catalysis, oxidation, and reduction can occur in these products. These reactions can arise from drug substance-excipient interactions, excipient-excipient interactions or container-product interactions. Particularly for pH sensitive compounds, these interactions may alter the pH and may decrease solubility and potentially cause precipitation.

Oxidative labile drug substances or vitamins, essential oils, and almost all fats and oils can be oxidized by auto-oxidation. Such reactions can be initiated by heat, light, peroxides, or other labile compounds or heavy metals such as copper or iron.

The effect of trace metals can be minimized by using chelating agents such as EDTA. Antioxidants may retard or delay oxidation by rapidly reacting with free radicals as they are formed (quenching). Common antioxidants include propyl, octyl and dodecylesters of gallic acid, butylated hydroxyanisole (BHA), butylated hydroxytoluene (BHT), ascorbic acid, sodium ascorbate, monothioglycerol, potassium or sodium metabisulfite, propionic acid, propyl gallate, sodium bisulfite, sodium sulfite, and the tocopherols or vitamin E.

In addition to stabilization of pharmaceutical preparations against chemical and physical degradation, liquid and semisolid preparations, particularly multiple dosed preparations, must usually be protected against microbial contamination. In contrast to solid preparations, aqueous solutions, syrups, emulsions, and suspensions often provide excellent growth media for microorganisms such as molds, yeast, and bacteria (e.g. *Pseudomonas Aeruginosa, E. Coli, Salmonella spp., Staphylococcus aureus, Candida albicans, Aspergillus niger*). Contamination by these microorganisms may occur during manufacturing or when a dose is taken from a multiple dosed formulation. Growth of the microorganisms occurs when a sufficient amount of water is present in the formulation.

Ophthalmic and injectable preparations are typically sterilized by autoclaving or filtration. However, many of them require the presence of an antimicrobial preservative to maintain aseptic conditions throughout their stated shelf life, specifically for multiple dosed preparations.

When a preservative is required, its selection is based upon several considerations, in particular the site of use whether internal, external or ophthalmic (for further details it can be referred to e.g. Remington, The Science and Practice of Pharmacy, 21st edition, Lippincott Williams & Wilkins, 2005).

Many liquid formulations for oral administration, particularly multiple dosed formulations, contain parabens as preservatives, e.g. methyl paraben (methyl-4-hydroxybenzoate) and propyl paraben (propyl-4-hydroxybenzoate). For example, in the Federal Republic of Germany liquid oral formulations containing parabens are commercialized under the trademarks: ben-u-ron®; Cetirizin-ratiopharm®; Pipamperon HEXAL®; Sedotussin®; TALOXA®; Truxal®; XUSAL®; talvosilen®; and Timonil®. Other commercialized liquid formulations contain sorbic acid or its potassium salt as preservative, e.g. ibuprofen liquid formulations and morphine liquid formulations.

Because of the number of excipients and additives in these preparations, it is recommended all the ingredients be listed on the container to reduce the risks that confront hypersensitive patients when these products are administered.

The preservatives benzalkonium chloride and potassium sorbate are also widely used e.g. in nasal drops and sprays. Recently, side effects resulting from mucosal damage caused by benzalkonium chloride and potassium sorbate were reported (cf. C.Y. Ho et al., Am J Rhinol. 2008, 22(2), 125-9). As far as hypersensitivity reactions of preservatives in topical ophthalmic therapies are concerned, quaternary ammoniums (benzalkonium chloride) are commonly associated with irritant toxic reactions whereas the organomercurials (thimerosal) and the alcohols (chlorobutanol) have high associations, respectively, with allergic responses (cf. J. Hong et al., Curr Opin Allergy Clin Immunol. 2009, 9(5), 447-53). Parabens have been implicated in numerous cases of contact sensitivity associated with cutaneous exposure (cf. M.G. Soni et al., Food Chem Toxicol. 2001, 39(6), 513-32) and have been reported to exert a weak estrogenic activity (cf. S. Oishi, Food Chem Toxicol. 2002, 40(12), 1807-13 and M.G. Soni et al., Food Chem Toxicol. 2005, 43(7), 985-015).

Due to these undesired side effects of known preservatives, it is desirable to provide aqueous pharmaceutical compositions (for example, for nasal administration) that exhibit a sufficient shelf life and in use stability in the absence of preservatives or at least in the presence of comparatively low quantities thereof.

WO 94/23711 A1 discloses stable solutions for the injection of ketamine, in particular the S(+) enantiomer, their preparation and use.

WO 2007/111880 A2 discloses compositions for the treatment of treatment-resistant depression, comprising intranasal administration of ketamine.

DE 10 2007 009888 A1 discloses S(+) ketamine for use in the treatment of depression, or its use in preparation of a medicament for the treatment of depression.

WO 2014/031975 A1 discloses a method for treating, ameliorating or preventing the onset of anxiety in a subject comprising administering to such subject an NMDA receptor antagonist in an amount that is sub-anesthetic and hypoanalgetic.

### SUMMARY OF THE INVENTION

The present invention is directed to a pharmaceutical composition of S-ketamine hydrochloride, comprising S-ketamine hydrochloride and water; wherein the pharmaceutical composition does not contain an antimicrobial preservative, wherein the S-ketamine hydrochloride is present in a concentration in the range of eq. 100 mg/mL to eq. 200 mg/mL, based on the total volume of the composition.

In an embodiment, the present invention is directed to a pharmaceutical composition of S-ketamine hydrochloride; wherein the formulation does not contain an antimicrobial preservative; and wherein the pharmaceutical composition is formulated for nasal administration.

### DETAILED DESCRIPTION OF THE INVENTION

The present invention is directed to pharmaceutical composition of S-ketamine, wherein the pharmaceutical composition does not contain an antimicrobial preservative, wherein the S-ketamine hydrochloride is present in a concentration of at least eq. 120 mg/mL, based on the total volume of the composition.

The pharmaceutical compositions of the present invention are based on the unexpected finding that S-ketamine hydrochloride exhibits preservative properties.

Thus, when formulating pharmaceutical compositions of S-ketamine hydrochloride, particularly aqueous liquid compositions, preservatives can be completely omitted while still achieving desired shelf life. Further, although the pharmaceutical compositions of S-ketamine hydrochloride of the present invention do not contain an antibacterial preservative, said compositions do not need to manufactured under aseptic conditions and / or do not need to be sterilized after production.

Additionally, wherein the pharmaceutical compositions of the present invention are formulated for nasal administration, the absence of preservatives results in the elimination of adverse effects associated with said preservatives, including for example, irritation or damage of the mucosal membrane.

As used herein, unless otherwise noted, the terms **"S-ketamine", "S-ketamine hydrochloride"** and **"esketamine"** shall mean the (S)-enantiomer of ketamine, as its corresponding hydrochloride salt, a compound of formula (I) 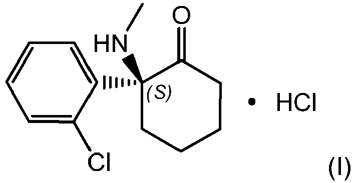 also known as (*S*)-2-(2-chlorophenyl)-2-(methylamino)cyclohexanone hydrochloride.

As used herein, the term **"composition"** is intended to encompass a product comprising the specified ingredients in the specified amounts, as well as any product which results, directly or indirectly, from combinations of the specified ingredients in the specified amounts.

The term **"pharmaceutical composition"** includes any pharmaceutical preparation or formulation that is customized for being administered to a human being or animal. Preferably, the composition contains one or more physiologically acceptable carriers and/or excipients. Preferably, the pharmaceutical compositions of the present invention contain water.

To prepare a pharmaceutical composition of the present invention, S-ketamine hydrochloride as the active ingredient is intimately admixed with a pharmaceutical carrier, preferably water, according to conventional pharmaceutical compounding techniques, which carrier may take a wide variety of forms depending of the form of preparation desired for administration. Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain. Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Also disclosed is an aqueous formulation of S-ketamine, comprising water and S-ketamine; wherein the S-ketamine is present in an amount in the range of from about 100 mg/mL to about 250 mg/mL, or any amount or range therein, based on the total volume of the pharmaceutical composition. Preferably, the S-ketamine is present in an amount in the range of from about 150 mg/ml to about 200 mg/mL, or any amount or range therein. More preferably, the S-ketamine is present in an amount in the range of from about 150 mg/mL to about 175 mg/mL, or any amount or range therein. More preferably, the S-Ketamine is present in an amount in the range of from about 160 mg/mL to about 163 mg/mL, for example, in an amount of about 161.4 mg/mL.

Also disclosed is an aqueous formulation of S-ketamine, comprising water and S-ketamine; wherein the S-ketamine is present in an amount in the range of from about eq. 100 mg/mL to about eq. 250 mg/mL, or any amount or range therein, based on the total volume of the pharmaceutical composition. Preferably, the S-ketamine is present in an amount in the range of from about eq. 125 mg/ml to about eq. 180 mg/mL, or any amount or range therein. More preferably, the S-ketamine is present in an amount in the range of from about eq. 140 mg/mL to about eq. 160 mg/mL, or any amount or range therein, for example, in an amount of about eq. 140 mg/mL.

The pharmaceutical compositions according to the invention are preferably an aqueous formulation. As used herein, unless otherwise noted, the term **"aqueous"** shall mean that the primary liquid component of the formulation is water. Preferably, water constitutes greater than about 80 wt% of the liquid component of the pharmaceutical composition, more preferably greater than about 90 wt%, more preferably greater than about 95 wt%, more preferably about 98 wt%.

In an embodiment of the present invention, the water content of the composition is within the range of 85±14 wt.-%, more preferably 85±12 wt.-%, still more preferably 85±10 wt.-%, most preferably 85±7.5 wt.-% and in particular 85±5 wt.-%, based on the total weight of the composition.

In another embodiment of the present invention, the water content of the composition is within the range of 90±14 wt.-%, more preferably 90±12 wt.-%, still more preferably 90±10 wt.-%, most preferably 80±7.5 wt.-% and in particular 90±5 wt.-%, based on the total weight of the composition.

In another embodiment of the present invention, the water content of the composition is within the range of 95±4.75 wt.-%, more preferably 95±4.5 wt.-%, still more preferably 95±4 wt.-%, yet more preferably 95±3.5 wt.-%, most preferably 95±3 wt.-% and in particular 95±2.5 wt.-%, based on the total weight of the composition.

In another embodiment of the present invention, the water content of the composition is within the range of from 75 to 99.99 wt.-%, more preferably 80 to 99.98 wt.-%, still more preferably 85 to 99.95 wt.-%, yet more preferably 90 to 99.9 wt.-%, most preferably 95 to 99.7 wt.-% and in particular 96.5 to 99.5 wt.-%, based on the total weight of the composition.

In another embodiment, the pharmaceutical compositions of the present invention further comprises one or more buffers and / or buffer systems (i.e. conjugate acid-base-pairs).

As used herein, the term **"buffer"** shall mean any solid or liquid composition (preferably an aqueous, liquid composition) which when added to an aqueous formulation adjusts the pH of said formulation. One skilled in the art will recognize that a buffer may adjust the pH of the aqueous formulation in any direction (toward more acidic, more basic or more neutral pH). Preferably, the buffer is pharmaceutically acceptable.

Suitably examples of buffers which may be used in the aqueous formulations of the present invention include, but are not limited to citric acid, sodium dihydrogen phosphate, disodium hydrogen phosphate, acetic acid, boric acid, sodium borate, succinic acid, tartaric acid, malic acid, lactic acid, furmaric acid, and the like. Preferably, the buffer or buffer system is selected from the group consisting of NaOH, citric acid, sodium dihydrogen phosphate and disodium hydrogen phosphate.

In an embodiment, the buffer is selected to adjust the pH of the S-ketamine hydrochloride pharmaceutical compositions of the present invention (e.g. the aqueous formulations described herein) into a pH in the range of from about pH 3.5 to about pH 6.5, or any amount or range therein. Preferably, the buffer is selected to adjust the pH of the S-ketamine hydrochloride compositions of the present invention to about in the range of from about pH 4.0 to about pH 5.5, or any amount or range therein, more preferably, in the range of from about pH 4.5 to about pH 5.0, or any amount or range therein.

Preferably, the concentration of the buffer and buffer system, respectively, preferably NaOH, is adjusted to provide a sufficient buffer capacity.

In an embodiment, the present invention is directed to a pharmaceutical composition comprising S-ketamine hydrochloride, water, and a buffer or buffer system, preferably NaOH; wherein the buffer or buffer system is present in an amount sufficient to yield a formulation with a pH in the range of from about pH 4.0 to about pH 6.0, or any amount or range therein.

The pharmaceutical compositions of the present invention do not contain a preservative.

As used herein, unless otherwise noted, the terms **"antimicrobial preservative"** and **"preservative"** preferably refer to any substance that is usually added to pharmaceutical compositions in order to preserve them against microbial degradation or microbial growth. In this regard, microbial growth typically plays an essential role, i.e. the preservative serves the main purpose of avoiding microbial contamination. As a side aspect, it may also be desirable to avoid any effect of the microbes on the active ingredients and excipients, respectively, i.e. to avoid microbial degradation.

Representative examples of preservatives include, but are not limited to, benzalkonium chloride, benzethonium chloride, benzoic acid, sodium benzoate, benzyl alcohol, bronopol, cetrimide, cetylpyridinium chloride, chlorhexidine, chlorbutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, sodium propionate, thimerosal, methyl paraben, ethyl paraben, propyl paraben, butyl paraben, isobutyl paraben, benzyl paraben, sorbic acid, and potassium sorbate.

The complete absence of preservatives in the pharmaceutical compositions of the present invention is preferred when the content of S-ketamine hydrochloride is sufficiently high so that due to its preservative property the desired shelf life or in use stability can be achieved by the presence of the drug itself. Under these circumstances the concentration of S-ketamine hydrochloride is at least eq. 120 mg/mL, preferably in the range of from about eq. 120 mg/mL to about eq. 175 mg/ml, or any amount or range therein, more preferably in an amount in the range of from about eq. 125 mg/mL to about eq. 150 mg/mL, or any amount or range therein, for example at about eq. 126 mg/mL or at about eq. 140 mg/mL.The pharmaceutical compositions of the present invention may further contain one or more additional excipients for example, wetting agents, surfactant components, solubilizing agents, thickening agents, colorant agents, antioxidant components, and the like.

Examples of a suitable antioxidant component, if used, include, but are not limited to one or more of the following: sulfites; ascorbic acid; ascorbates, such as sodium ascorbate, calcium ascorbate, or potassium ascorbate; ascorbyl palmitate; fumaric acid; ethylene diamine tetraacetic acid (EDTA) or its sodium or calcium salts; tocopherol; gallates, such as propyl gallate, octyl gallate, or dodecyl gallate; vitamin E; and mixtures thereof. The antioxidant component provides long term stability to the liquid compositions. Addition of the antioxidant component can help enhance and ensure the stability of the compositions and renders the compositions stable even after six months at 40 °C. A suitable amount of the antioxidant component, if present, is about 0.01 wt.-% to about 3 wt.-%, preferably about 0.05 wt.-% to about 2 wt.-%, of the total weight of the composition.

Solubilizing and emulsifying agents can be included to facilitate more uniform dispersion of the active ingredient or other excipient that is not generally soluble in the liquid carrier. Examples of a suitable emulsifying agent, if used, include, but are not limited to, for example, gelatin, cholesterol, acacia, tragacanth, pectin, methyl cellulose, carbomer, and mixtures thereof. Examples of a suitable solubilizing agent include polyethylene glycol, glycerin, D-mannitol, trehalose, benzyl benzoate, ethanol, trisaminomethane, cholesterol, triethanolamine, sodium carbonate, sodium citrate, sodium salicylate, sodium acetate, and mixtures thereof.

Preferably, the solubilizing agent includes glycerin. The solubilizing or emulsifying agent is/are generally present in an amount sufficient to dissolve or disperse the active ingredient, i.e. S-ketamine, in the carrier. Typical amounts when a solubilizing or an emulsifier are included are from about 1 wt.-% to about 80 wt.-%, preferably about 20 wt.-% to about 65 wt.-%, and more preferably about 25 wt.-% to about 55 wt.-%, of the total weight of the composition.

A suitable isotonizing agent, if used, includes sodium chloride, glycerin, D-mannitol, D-sorbitol, glucose, and mixtures thereof. A suitable amount of the isotonizing agent, when included, is typically about 0.01 wt.-% to about 15 wt.-%, more preferably about 0.3 wt.-% to about 4 wt.-%, and more preferably about 0.5 wt.-% to about 3 wt.-%, of the total weight of the composition.

A Suspending agent or viscosity increasing agent can be added to to the pharmaceutical compositions of the present invention, to for example, increase the residence time in the nose. Suitably examples include, but are not limited to, hydroxypropyl methylcellulose, sodium carmellose, microcrystalline cellulose, carbomer, pectin, sodium alginate, chitosan salts, gellan gum, poloxamer, polyvinyl pyrrolidone, xanthan gum, and the like.

As used herein the term **"shelf life"** refers to the storage stability of a closed container of the pharmaceutical composition.

Preferably, the pharmaceutical compositions according to the present invention exhibit antimicrobial robustness that complies with the requirements for nasal pharmaceutical compositions (for example, the Ph. Eur. requirements). Requirements for the following organisms: Staphylococcus aureus, Escherichia coli, Pseudomonas aeruginosa, Candida albicans, Aspergillus niger (for example, A. Brasiliensis, a niger variety), and Micrococcus luteus (an in-house, J&J organism) are as listed in Table 1, below.

**Table 1: Bacterial & Fungal Requirements for Nasal Compositions**

| **Bacteria** | | | | | | |
|---|---|---|---|---|---|---|
| | **6 hrs** | **24 hrs** | **48 hrs** | **7 days** | **14 days** | **28 days** |
| **Requirement Std.** | | | | | | |
| Eur. A | - | - | log 2 | log 3 | - | NI/7d |
| Eur. B | - | - | - | - | log 3 | NI/14d |
| J&J | - | - | - | - | log 3 | NI/14d |
| U.S.P. | - | - | - | - | >= 2.0 | NI/14d |
| J.P. | - | - | - | - | >= 2.0 | NI/14d |

| **Fungi** | | | | | | |
|---|---|---|---|---|---|---|
| **Requirement Std.** | **6 hrs** | **24 hrs** | **48 hrs** | **7 days** | **14 days** | **28 days** |
| Eur. A | - | - | - | - | log 2 | NI/14d |
| Eur. B | - | - | - | - | log 1 | NI/14d |
| J.&J. | - | - | - | - | log 2.0 | NI/14d |
| U.S.P. | - | - | - | - | NI/Init. | NI/Init. |
| J.P. | - | - | - | - | NI/Init. | NI/Init. |

Preferably, antimicrobial robustness is achieved against one or more of *E. coli, S. aureus, Ps. Aeruginosa, S. spp., M. luteus and* / *or C. albicans.*

Preferably, the pharmaceutical compositions of the present invention exhibit a shelf-life under accelerated storage conditions of at least 1 month, more preferably at least 2 months, still more preferably at least 3 months, yet more preferably at least 4 months, most preferably at least 5 months and in particular at least 6 months. Preferably, the shelf life is determined according to Ph. Eur., particularly as described in the experimental section. Accelerated storage conditions preferably mean 40°C / 75% Relative Humidity (%RH).

Preferably, the pharmaceutical compositions of the present invention exhibit a shelf-life under ambient conditions of at least 6 months, more preferably at least 12 months, still more preferably at least 15 months, yet more preferably at least 18 months, most preferably at least 21 months and in particular at least 24 months.

In an embodiment, the present invention is directed to a pharmaceutical composition comprising: (a) S-ketamine hydrochloride @ 161.4 mg/mL; (b) NaOH q.s. ad pH 4.5; wherein the NaOH is preferably added to the pharmaceutical composition as a 1N solution; (c) purified water q.s. ad 1000 µL; and wherein pharmaceutical compositions does not contain antimicrobial preservative.

In an embodiment, the pharmaceutical composition of the present invention is prepared by adding water to the S-ketamine hydrochloride; followed by addition of 1N NaOH_{(aq)} to adjust the pH of the resulting mixture to the desired pH, preferably to a pH in the range of from about pH 3.5 to about pH 6.0, more preferably to about pH in the range of from about pH 4.0 to about pH 5.0, more preferably to about pH 4.5.

In a preferred embodiment, the pharmaceutical compositions of the present invention are ready to use, i.e. do not require particular treatment steps such as dissolution in a solvent before they may be administered to the patient.

A skilled person recognizes that the pharmaceutical compositions of the present invention may alternatively be commercialized as a precursor in form of a dry powder that is to be dissolved or dispersed in an appropriate amount of water prior to the first use.

In an embodiment, the present invention is directed to a pharmaceutical composition (S-ketamine HCl eq. 140 mg/mL) comprising S-ketamine HCl, citric acid (preferably citric acid 1 aqua), disodium edetate, sodium hydroxide and water.

In another embodiment, the present invention is directed to a pharmaceutical composition (S-ketamine HCl eq. 140 mg/mL) comprising, consisting of or consisting essentially of the following components in the listed amounts:

| | | |
|---|---|---|
| S-Ketamine Hydrochloride | = | about 161.4 mg/ml |
| Citric acid 1 aqua | = | about 1.5 mg/ml |
| Disodium edetate | = | about 0.12 mg/ml |
| Sodium hydroxide all use | = | q.s. ad pH 4.5 |
| Water for Injection | = | q.s. ad 1 ml |

q.s. ad = sufficient amount to attain desired criteria (e.g. pH, injection volume).

A further aspect of the invention relates to a pharmaceutical dosage form comprising the pharmaceutical composition according to the invention. All preferred embodiments that are described above in connection with the composition according to the invention also apply to the dosage form according to the invention.

In an embodiment, the dosage form according to the invention is adapted for nasal administration. Preferably, the dosage form according to the invention is adapted for administration once every couple of days, on individually determined days only; or once daily, twice daily, thrice daily, four times daily, five times daily, six times daily or even more frequently; or clustered as between 2 and up to 8 consecutive administrations within a limited time period ranging from 1 to about 60 minutes.

The present invention is further directed to a pharmaceutical composition or a pharmaceutical dosage form for use in methods for the treatment of depression, preferably resistant depression or treatment refractory depression, comprising administering to a subject in need thereof, a therapeutically effective amount of any of the pharmaceutical compositions a described herein. Preferably, the administration is nasal.

In an embodiment, the present invention is directed to a pharmaceutical composition or a pharmaceutical dosage form for use in a method for the treatment of depression, preferably resistant depression or treatment refractory depression, comprising the nasal administration of the pharmaceutical composition according to the invention as described above or of the pharmaceutical dosage form according to the invention as described above, to a subject in need thereof.

Major Depressive Disorder is defined as the presence of one of more major depressive episodes that are not better accounted for psychotic disorder or bipolar disorder. A major depressive episode is characterized by meeting five or more of the following criteria during the same 2 week period which represent a change in functioning and include at least depressed/ sad mood or loss of interest and pleasure, indifference or apathy, or irritability and is usually associated with a change in a number of neurovegetative functions, including sleep patterns, appetite and body weight, motor agitation or retardation, fatigue, impairment in concentration and decision making, feelings of shame or guilt, and thoughts of death or dying (Harrison's Principles of Internal Medicine, 2000). Symptoms of a depressive episode include depressed mood; markedly diminished interest or pleasure in all, or almost all, activities most of the day; weight loss when not dieting or weight gain, or decrease or increase in appetite nearly every day; insomnia or hypersomnia nearly every day; psychomotor agitation or retardation nearly every day; fatigue or loss of energy nearly every day; feelings of worthlessness or excessive or inappropriate guilt nearly every day; diminished ability to think or concentrate, or indecisiveness, nearly every day; recurrent thoughts of death, recurrent suicidal ideation without a specific plan, or a suicide attempt or a specific plan for committing suicide. Further, the symptoms cause clinically significant distress or impairment in social, occupational, or other important areas of functioning. (Diagnostic and Statistical Manual of Mental Disorders, 4th Edition, American Psychiatric Association, 1994)

As used herein, the term **"depression"** shall be defined to include major depressive disorder, unipolar depression, treatment-refractory depression, resistant depression, anxious depression, bipolar depression and dysthymia (also referred to as dysthymic disorder). Preferably, the depression is major depressive disorder, unipolar depression, treatment-refractory depression, resistant depression, anxious depression or bipolar depression.

As used herein, the term **"treatment-refractory or treatment-resistant depression"** and the abbreviation **"TRD"** shall be defined as major depressive disorder that does not respond to adequate courses of at least two antidepressants, preferably two or more antidepressants, more preferably two to three, antidepressants.

As used herein, the term **"bipolar depression"** is intended to mean the depression associated with, characteristic of or symptomatic of a bipolar disorder. Thus, methods of treating bipolar depression of the present invention are directed to methods which treat the depression and / or depressed phase of bipolar disorders.

One skilled in the art will recognize that the failure to respond to an adequate course of a given antidepressant may be determined retrospectively or prospectively. In an embodiment, at least one of the failures to respond to an adequate course of antidepressant is determined prospectively. In another embodiment, at least two of the failures to respond to an adequate course of antidepressant are determined prospectively. In another embodiment, at least one of the failures to respond to an adequate course of antidepressant is determined retrospectively. In another embodiment, at least two of the failures to respond to an adequate course of antidepressant are determined retrospectively.

As used herein, unless otherwise noted, the terms **"treating", "treatment"** and the like, shall include the management and care of a subject or patient (preferably mammal, more preferably human) for the purpose of combating a disease, condition, or disorder and includes the administration of a compound of the present invention to prevent the onset of the symptoms or complications, alleviate the symptoms or complications, or eliminate the disease, condition, or disorder.

The term **"therapeutically effective amount"** as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

Optimal dosages to be administered may be readily determined by those skilled in the art, and will vary with the particular compound used, the mode of administration, the strength of the preparation, the mode of administration, the number of consecutive administrations within a limited period of time (e.g. up to 60 minutes) and the advancement of the disease condition. In addition, factors associated with the particular patient being treated, including patient age, weight, diet and time of administration, will result in the need to adjust dosages.

The term **"subject"** as used herein, refers to an animal, preferably a mammal, most preferably a human, who has been the object of treatment, observation or experiment. Preferably, the subject has experienced and / or exhibited at least one symptom of the disease or disorder to be treated and / or prevented.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The following Examples are set forth to aid in the understanding of the invention, and are not intended and should not be construed to limit in any way the invention set forth in the claims which follow thereafter.

### Example 1: Microbial Challenge Test for Nasal Spray Pharmaceutical Composition Containing S-ketamine Hydrochloride (eg. 140 mg/ml)

An aqueous formulation of S-ketamine hydrochloride referred to as "S-ketamine eq. 140 mg/ml below, was prepared by mixing S-ketamine hydrochloride (at a concentration of 161.4 mg/ml) in water and then adding 1N NaOH_{(aq)} to pH 5.0.

A challenge test was initiated to investigate whether the formulation could prevent microorganisms from proliferating. The challenge test consisted of challenging the formulation with a prescribed inoculum of microorganisms. The inoculated formulation was then stored at room temperature and at specified time intervals a sample was withdrawn to count the microorganisms in the sample.

As shown in Table 2 below, the challenge test results on S-ketamine eq. 140 mg/ml pH 5.0 show that S-ketamine eq. 140mg/ml pH 5.0 reduced the original spike (10⁵-10⁶ CFU/ml) of bacteria (i.e. *Pseudomonas aeruginosa, Staphylococcus aureus, Escherichia coli* and environmental isolate *Staphylococcus haemolyticus*) and also of the yeast *Candida albicans.* S-ketamine eq. 140mg/ml pH 5.0 was not able to reduce the original spike of *Aspergillus brasiliensis* to the same extent as for the bacteria and yeast, but no increase was observed after 28 days of incubation at room temperature.

**Table 2: Challenge Test S-ketamine eg. 140 mg/ml pH 5.0 (CFU/ml)**

| **Organism** | **Blank at 0 hours** | **Product** | | | | |
|---|---|---|---|---|---|---|
| | | **at 0 hours** | **at 2 days** | **at 7 days** | **at 14 days** | **at 28 days** |
| *A. brasiliensis* | 1.50 x 10⁵ | 1.90 x 10⁵ | ND | 4.80 x 10⁴ | 5.80 x 10⁴ | 2.65 x 10⁴ |
| *C. albicans* | 1.15 x 10⁵ | 6.50 x 10⁴ | ND | < 5 | < 5 | < 5 |
| *P. aeruginosa* | 2.65 x 10⁵ | 1.59 x 10⁴ | < 50 | < 5 | < 5 | < 5 |
| *S. aureus* | 1.90 x 10⁵ | 1.70 x 10⁵ | < 50 | < 5 | < 5 | < 5 |
| *E. coli* | 1.20 x 10⁵ | 6.00 x 10⁴ | < 50 | < 5 | < 5 | < 5 |
| *S. haemolyticus* | 1.20 x 10⁵ | 7.50 x 10⁴ | < 50 | < 5 | < 5 | < 5 |

| | | | | | | |
|---|---|---|---|---|---|---|
| ND: not determined | | | | | | |

### Example 2: Microbial Challenge for Nasal Spray Pharmaceutical Composition Containing S-ketamine Hydrochloride

Aqueous formulation of S-ketamine hydrochloride as listed in Table 3, below, were prepared by mixing S-ketamine hydrochloride (at the listed concentrations) in water and then adding 1N NaOH_{(aq)} to the listed pH levels.

**Table 3: Composition of edge of failure batches**

| **Formulation** | **Concentration API(mg/ml)** | **pH** |
|---|---|---|
| F-1 | eq. 126 | 4.0 |
| F-2 | eq. 140 | 4.5 |
| F-3 | eq. 126 | 5.0 |
| F-4 | eq. 126 | 4.5 |

### Low Level A brasiliensis Challenge

The formulations listed in Table 3, above were subjected to a low level challenge with *A. brasiliensis* to evaluate whether the formulations would be able to reduce this lower spike, with results as shown in Table 4, below. A spike of 10³ CFU/ml was chosen instead of 10⁵-10⁶ CFU/ml, which is the standard spike for a challenge test.

**Table 4: Low Level Challenge with A. brasiliensis**

| | **Product (CFU/ml)** | | | |
|---|---|---|---|---|
| **Formulation** | **0 hours** | **7 days** | **14 days** | **28 days** |
| F-1 | 4.45 E+02 | 2.35 E+02 | 2.15 E+02 | 1.75 E+02 |
| F-2 | 3.80 E+02 | 2.50 E+02 | 2.00 E+02 | 2.00 E+02 |
| F-3 | 5.50 E+02 | 2.75 E+02 | 2.65 E+02 | 1.10 E+02 |
| F-4 | 3.85 E+02 | 2.70 E+02 | 2.60 E+02 | 1.55 E+02 |
| Blank | 4.75 E+02 | NA | NA | NA |

The results, as listed in Table 3, above, show that in none of the tested formulation was an increase in *Aspergillus brasiliensis* observed after 28 days of incubation, rather a minor and slow decrease was observed.

### Full AET Challenge Test

The formulations listed in Table 3 were additionally subjected to a Full AET challenge, with results for the individual formulations are shown in Tables 5-8, below. Additionally, Table 9 below, provides results for a Full AET challenge of a reference formulation containing 0.00 mg/mL S-ketamine hydrochloride, denatonium benzoate (to mimic the taste of the S-ketamine HCl formulation(s)), and adjusted to pH 5.21 with 1N NaOH.

**Table 5: Full AET Challenge Test**

| **Formulation F-1: S-ketamine eq. 126 mg/ml pH 4.0 (CFU/ml)** | | | | | | |
|---|---|---|---|---|---|---|
| **Organism** | **Blank at 0 hrs** | **Product** | | | | |
| | | **at 0 hrs** | **at 2 days** | **at 7 days** | **at 14 days** | **at 28 days** |
| *A. brasiliensis* | 1.55 x 10⁵ | 2.15 x 10⁵ | ND | 5.30 x 10⁴ | 2.10 x 10⁴ | 3.5 x 10³ |
| *C. albicans* | 1.05 x 10⁵ | 1.15 x 10⁴ | ND | < 5 | < 5 | < 5 |
| *P. aeruginosa* | 5.05 x 10⁵ | 1.10 x 10⁴ | < 50 | < 5 | < 5 | < 5 |
| *S. aureus* | 6.00 x 10⁵ | 6.25 x 10⁵ | < 50 | < 5 | < 5 | < 5 |
| *E. coli* | 6.05 x 10⁵ | 4.00 x 10⁴ | < 50 | < 5 | < 5 | < 5 |
| *M. luteus* | 1.65 x 10⁵ | 7.50 x 10⁴ | < 50 | < 5 | < 5 | < 5 |

| | | | | | | |
|---|---|---|---|---|---|---|
| ND: not determined | | | | | | |

**Table 6: Full AET Challenge Test**

| **Formulation F-2: S-ketamine eq. 140 ma/ml pH 4.5 (CFU/ml)** | | | | | | |
|---|---|---|---|---|---|---|
| **Organism** | **Blank at 0 hrs** | **Product** | | | | |
| | | **at 0 hrs** | **at 2 days** | **at 7 days** | **at 14 days** | **at 28 days** |
| *A. brasiliensis* | 1.55 x 10⁵ | 1.20 x 10⁵ | ND | 1.65 x 10⁴ | 1.00 x 10⁴ | 2.0 x 10³ |
| *C. albicans* | 1.05 x 10⁵ | 1.15 x 10⁴ | ND | < 5 | < 5 | < 5 |
| *P. aeruginosa* | 5.05 x 10⁵ | 7.55 x 10⁴ | < 50 | < 5 | < 5 | < 5 |
| *S. aureus* | 6.00 x 10⁵ | 5.70 x 10⁵ | < 50 | < 5 | < 5 | < 5 |
| *E. coli* | 6.05 x 10⁵ | 2.80 x 10⁴ | < 50 | < 5 | < 5 | < 5 |
| *M. luteus* | 1.65 x 10⁵ | 1.70 x 10⁴ | < 50 | < 5 | < 5 | < 5 |

| | | | | | | |
|---|---|---|---|---|---|---|
| ND: not determined | | | | | | |

**Table 7: Full AET Challenge TestFormulation F-3: S-ketamine eq. 126 mg/ml pH 5.0 (CFU/ml)**

| **Formulation F-3: S-ketamine eq. 126 mg/ml pH 5.0 (CFU/ml)** | | | | | | |
|---|---|---|---|---|---|---|
| **Organism** | **Blank at 0 hrs** | **Product** | | | | |
| | | **at 0 hrs** | **at 2 days** | **at 7 days** | **at 14 days** | **at 28 days** |
| *A. brasiliensis* | 1.55 x 10⁵ | 1.90 x 10⁵ | ND | 3.50 x 10⁴ | 2.15 x 10⁴ | 8.0 x 10³ |
| *C. albicans* | 1.05 x 10⁵ | 1.60 x 10⁴ | ND | < 5 | < 5 | < 5 |
| *P. aeruginosa* | 5.05 x 10⁵ | 1.03 x 10⁴ | < 50 | < 5 | < 5 | < 5 |
| *S. aureus* | 6.00 x 10⁵ | 6.05 x 10⁵ | < 50 | < 5 | < 5 | < 5 |
| *E. coli* | 6.05 x 10⁵ | 2.00 x 10⁴ | < 50 | < 5 | < 5 | < 5 |
| *M. luteus* | 1.65 x 10⁵ | 1.60 x 10⁴ | < 50 | < 5 | < 5 | < 5 |

| | | | | | | |
|---|---|---|---|---|---|---|
| ND: not determined | | | | | | |

**Table 8: Full AET Challenge Test**

| **Formulation F-4: S-ketamine eq. 126 mg/ml pH 4.5 (CFU/ml)** | | | | | | |
|---|---|---|---|---|---|---|
| **Organism** | **Blank at 0 hrs** | **Product** | | | | |
| | | **at 0 hrs** | **at 2 days** | **at 7 days** | **at 14 days** | **at 28 days** |
| *A. brasiliensis* | 1.55 x 10⁵ | 2.10 x 10⁵ | ND | 5.30 x 10⁴ | 2.10 x 10⁴ | 5.5 x 10³ |
| *C. albicans* | 1.05 x 10⁵ | 2.85 x 10⁴ | ND | < 5 | < 5 | < 5 |
| *P. aeruginosa* | 5.05 x 10⁵ | 8.35 x 10⁴ | < 50 | < 5 | < 5 | < 5 |
| *S. aureus* | 6.00 x 10⁵ | 7.60 x 10⁵ | < 50 | < 5 | < 5 | < 5 |
| *E. coli* | 6.05 x 10⁵ | 4.80 x 10⁴ | < 50 | < 5 | < 5 | < 5 |
| *M. luteus* | 1.65 x 10⁵ | 7.00 x 10⁴ | < 50 | < 5 | < 5 | < 5 |

| | | | | | | |
|---|---|---|---|---|---|---|
| ND: not determined | | | | | | |

**Table 9: Full AET Challenge Test**

| **Reference Formulation: 0.0 mg/mL S-ketamine, Denatonium Benzoate (to mimic S-ketamine HCl taste), pH 5.21** | | | | | | |
|---|---|---|---|---|---|---|
| **Organism** | **Blank at 0 hours** | **At 0 hours** | **At 2 days** | **At 7 days** | **At 14 days** | **At 28 days** |
| *A. Brasileinsis* | 1.55 x 10⁵ | 1.75 x 10⁵ | - | 4.35 x 10⁴ | 8.00 x 10⁴ | 9.00 x 10⁴ |
| *C. Albicans* | 1.05 x 10⁵ | 1.20 x 10⁵ | - | 1.02 x 10⁵ | 1.40 x 10⁵ | 1.30 x 10⁵ |
| *P. aeruginosa* | 5.05 x 10⁵ | 4.05 x 10⁵ | 6.55 x 10⁵ | >2.00 x 10⁵ | 9.75 x 10⁵ | 2.40 x 10⁶ |
| *S. aureus* | 6.00 x 10⁵ | 5.15 x 10⁵ | 3.90 x 10⁵ | 1.55 x 10² | <5 | <5 |
| *E. coli* | 6.05 x 10⁵ | 5.55 x 10⁵ | 6.40 x 10⁵ | >2.00 x 10⁵ | 8.25 x 10⁵ | 1.09 x 10⁶ |
| *M. luteus* | 1.65 x 10⁵ | 6.00 x 10⁴ | 8.50 x 10⁴ | 4.30 x 10² | 2.50 x 10³ | 3.90 x 10² |

As shown in Tables 5-10 above, after 28 days, all the tested formulations reduced the original spike (10⁵-10⁶ CFU/ml) of bacteria (i.e. *Pseudomonas aeruginosa, Staphylococcus aureus, Escherichia coli* and environmental isolate *Staphylococcus haemolyticus*) and environmental *Micrococcus Luteus,* and also of the yeast *Candida albicans.* The tested formulations were not able to reduce the original spike of *Aspergillus brasiliensis* to the same extent as for the bacteria and yeast, but no increase was observed after 28 days of incubation at room temperature.

In summary, the results presented in Biological Example 1 and Biological Example 2 indicated that S-ketamine hydrochloride exhibits strong antimicrobial properties against *Pseudomonas aeruginosa, Staphylococcus aureus, Escherichia coli,* environmental isolate *Staphylococcus haemolyticus,* environmental *Micrococcus Luteus,* and the yeast *Candida albicans.* For *Aspergillus brasiliensis* the decrease in microorganisms is less pronounced, but no increase was observed, indicating some inhibitory activity.

### Example 3

### S-ketamine HCl Formulation Microbial Challenge

An S-ketamine HCl nasal formulation was prepared, comprising the components listed below.

| **Excipient** | **Concentration** |
|---|---|
| S-Ketamine Hydrochloride | = 161.4 mg/mL |
| Citric acid 1 aqua | = 1.5 mg/mL |
| Disodium edetate | = 0.12 mg/mL |
| Sodium hydroxide all use | = q.s. ad pH 4.5 |
| Water for Injection | = q.s. ad 1 ml |

Applied culture media and diluent were prepared as follows. Tryptic Soy Agar (TSA) was prepared as a mixture of peptone from casein 15.0 g/L, peptone from soymeal 5.0 g/Lm sodium chloride 5.0 g/l and agar-agar, 15.0 g/L. Sabouraud Detrose Agar (SAB) was prepared as a mixture of peptone 10.0 g/L, D(+)glucose, 40.0 g/L and agar-agar, 15.0 g/L. Modified Letheen Broth was prepared as a mixture of peptone from casein, 15.0 g/L, peptone from meat, 10.0 g/L, meat extract, 5.0 g/L, yeast extract, 2.0 g/L, sodium chloride, 10.0 g/L, lecithin, 0.7 g/L, sodium bisulfite, 0.1 g/L and polysorbate 80, 5.0 g/L. The following Inoculum suspensions were prepared for testing:

| **Organism** | **Suspension (CFU / mL)** |
|---|---|
| *Aspergillus brasiliensis* | 1.7 X 10⁷ |
| *Candida albicans* | > 1.00 X 10⁸ |
| *Pseudomonas aeruginosa* | > 1.00 X 10⁸ |
| *Staohylococcus aureus* | > 1.00 X 10⁸ |
| *Escherichia coli* | > 1.00 X 10⁸ |
| *S. arlettae* | > 1.00 X 10⁸ |

Three batches of the formulation described above were tested against the inoculum suspensions listed above, with results after 0 hours, 2 days, 7 days, 14 days and 28 days, with results as listed in Tables 10, 11 and 12 below.

**Table 10: Batch 1: Antimicrobial Efficacy Results**

| | **Blank** | **Formulation** | | | | |
|---|---|---|---|---|---|---|
| **Organism** | **0 hrs** | **0 hrs** | **2 days** | **7 days** | **14 days** | **28 days** |
| *A. brasiliensis* | 1.00 X 10⁵ | 1.10 X 10⁵ | - | 2.05 X 10⁴ | 2.15 X 10⁴ | 5.50 X 10³ |
| *C albicans* | 1.30 X 10⁵ | 1.30 X 10⁵ | - | < 10 | < 10 | < 10 |
| *P. aeruginosa* | 7.15 X 10⁵ | 5.50 X 10⁵ | < 100 | < 10 | < 10 | < 10 |
| *S. aureus* | 5.30 X 10⁵ | 4.30 X 10⁵ | < 100 | < 10 | < 10 | < 10 |
| *E. coli* | 4.10 X 10⁵ | 2.90 X 10⁵ | < 100 | < 10 | < 10 | < 10 |
| *S*. *arlettae* | 1.55 X 10⁵ | 1.30 X 10⁵ | < 100 | < 10 | < 10 | < 10 |

| | | **Negative Control - Tryptic Soy Agar (TSA)** | | | | |
|---|---|---|---|---|---|---|
| | | **0 hrs** | **2 days** | **7 days** | **14 days** | **28 days** |
| | | < 10 | < 10 | < 10 | < 10 | < 10 |

| | | **Negative Control - Sabouraud Dextrose Agar (SDA)** | | | | |
|---|---|---|---|---|---|---|
| | | **0 hrs** | **2 days** | **7 days** | **14 days** | **28 days** |
| | | < 10 | < 10 | < 10 | < 10 | < 10 |

**Table 11: Batch 2: Antimicrobial Efficacy Results**

| | **Blank** | **Formulation** | | | | |
|---|---|---|---|---|---|---|
| **Organism** | **0 hrs** | **0 hrs** | **2 days** | **7 days** | **14 days** | **28 days** |
| *A. brasiliensis* | 1.00 X 10⁵ | 1.15 X 10⁵ | - | 1.90 X 10⁴ | 9.50 X 10³ | 1.00 X 10³ |
| *C albicans* | 1.30 X 10⁵ | 1.85 X 10⁵ | - | 1.15 X 10² | < 10 | < 10 |
| *P. aeruginosa* | 7.15 X 10⁵ | 3.65 X 10⁵ | < 100 | < 10 | < 10 | < 10 |
| *S*. *aureus* | 5.30 X 10⁵ | 5.15 X 10⁵ | < 100 | < 10 | < 10 | < 10 |
| *E. coli* | 4.10 X 10⁵ | 3.35 X 10⁵ | < 100 | < 10 | < 10 | < 10 |
| *S*. *arlettae* | 1.55 X 10⁵ | 1.40 X 10⁵ | < 100 | < 10 | < 10 | < 10 |

| | | **Negative Control - Tryptic Soy Agar (TSA)** | | | | |
|---|---|---|---|---|---|---|
| | | **0 hrs** | **2 days** | **7 days** | **14 days** | **28 days** |
| | | < 10 | < 10 | < 10 | < 10 | < 10 |

| | | **Negative Control - Sabouraud Dextrose Agar (SDA)** | | | | |
|---|---|---|---|---|---|---|
| | | **0 hrs** | **2 days** | **7 days** | **14 days** | **28 days** |
| | | < 10 | < 10 | < 10 | < 10 | < 10 |

**Table 12: Batch 3: Antimicrobial Efficacy Results**

| | **Blank** | **Formulation** | | | | |
|---|---|---|---|---|---|---|
| **Organism** | **0 hrs** | **0 hrs** | **2 days** | **7 days** | **14 days** | **28 days** |
| *A. brasiliensis* | 1.00 X 10⁵ | 9.50 X 10⁴ | - | 2.65 X 10⁴ | 1.90 X 10⁴ | 1.00 X 10³ |
| *C albicans* | 1.30 X 10⁵ | 2.20 X 10⁵ | - | 3.50 X 10¹ | < 10 | < 10 |
| *P. aeruginosa* | 7.15 X 10⁵ | 4.45 X 10⁵ | < 100 | < 10 | < 10 | < 10 |
| *S*. *aureus* | 5.30 X 10⁵ | 4.50 X 10⁵ | < 100 | < 10 | < 10 | < 10 |
| *E. coli* | 4.10 X 10⁵ | 2.60 X 10⁵ | < 100 | < 10 | < 10 | < 10 |
| *S*. *arlettae* | 1.55 X 10⁵ | 1.40 X 10⁵ | < 100 | < 10 | < 10 | < 10 |

| | | **Negative Control - Tryptic Soy Agar (TSA)** | | | | |
|---|---|---|---|---|---|---|
| | | **0 hrs** | **2 days** | **7 days** | **14 days** | **28 days** |
| | | < 10 | < 10 | < 10 | < 10 | < 10 |

| | | **Negative Control - Sabouraud Dextrose Agar (SDA)** | | | | |
|---|---|---|---|---|---|---|
| | | **0 hrs** | **2 days** | **7 days** | **14 days** | **28 days** |
| | | < 10 | - | < 10 | < 10 | < 10 |

### Example 4

### 6-12 Month Stability Evaluation, S-Ketamine Nasal Formuation

An S-ketamine HCl nasal formulation was prepared, comprising the components listed below.

| **Excipient** | **Concentration** |
|---|---|
| S-Ketamine Hydrochloride | = 161.4 mg/mL |
| Sodium Hydroxide | = q.s. ad pH 4.5 |
| Water for Injection | = q.s. ad 1 ml |

The stability of the S-ketamine HCl eq. 140 mg/mL formulation described above, packed in a Type 1 clear glass vial assembled into a nasal spray device (stored in a horizontal position) was evaluated through 12 months at long term and accelerated conditions (12 months at 25°C/40%RH and 6 months at 40°C/20%RH). The container comprised a glass vial (MGlas AG Bi-Dose Vial, FIOLAX glass, Type 1), plunger (West Pharma Resin PH 4432/50, chlorobutyl elastomer formulation, gray) and device (Aptar Container Holder PP Natural and Aptar Actuator, Actuator_ASM, Nasal Adult, ADD, white). The following test parameters (and test methods) were used in the evaluation:
(a) Droplet size distribution (laser diffraction);
(b) Spray pattern (laser diffraction, high speed photographic recording);
(c) Actuation force (force profile recording);
(d) Plume geometry (high speed photographic recording);
(e) Rest volume (Current USP <1151> / based on Ph. Eur. 2.9.17); and
(f) Osmolality (Current USP <785> / based on Ph. Eur. 2.2.35).

Testing of these parameters was evaluated according to the schedule listed in Table 13, below.

**Table 13: Parameters Tested and Timing**

| **Time** | **Long Term (25°C/40%RH)** | **Accelerated (40°C/20%RH)** |
|---|---|---|
| 0 (Initial) | a, b, c, d, e, f, | Not tested |
| 3 mo. | a, b, c | a, b |
| 6 mo. | a, b, c | a, b |
| 12 mo. | a, b, c, d | Not tested |

### Results:

**Droplet size distribution:** No substantial stability related changes in droplet size distribution at 3 cm and 6 cm laser height were observed during storage at the different storage conditions. **Spray pattern:** No substantial stability related changes in spray pattern at 3 cm and 6 cm laser height were observed during storage at the different storage conditions. **Actuation force:** No substantial stability related changes in actuation force were observed during storage at the different storage conditions. **Plume geometry:** At the initial time point no deviations in plume geometry were observed. **Rest volume:** At the initial time point no deviations in rest volume were observed. **Osmolality:** At the initial time point no deviations in osmolality were observed.

### Example 5

### 6-12 Month Stability Evaluation, S-Ketamine Nasal Formulation

An S-ketamine HCl nasal formulation was prepared, comprising the components listed below.

| **Excipient** | **Concentration (mg/ml)** |
|---|---|
| S-Ketamine Hydrochloride | = 161.4 mg/mL |
| Sodium Hydroxide | = q.s. ad pH 4.5 |
| Water for Injection | = q.s. ad 1 ml |

The stability of the S-ketamine HCl eq. 140 mlg/mL formulation, packed in clear glass vials or in devices, was evaluated through 12 months at long term (25°C/40%RH) and 6 months at accelerated conditions (40°C/25%RH). The devices comprised a glass vial (MGlas AG Bi-Dose Vial, FIOLAX glass, Type 1, clear), plunger (West Pharma Resin PH 4432/50, chlorobutyl elastomer formulation, gray) and device (Container holder, PP, Natural and Actuator_ASM, nasal adult, white). The following test parameters (and test methods) were used in the evaluation:
(a) Appearance (Visual examination);
(b) Assay of S-Ketamine HCl (Ultra Performance Liquid Chromatography (UPLC));
(c) Chromatographic purity (UPLC);
(d) pH (Current USP <791> / based Ph. Eur. 2.2.3);
(e) Stereo-isomeric purity (HPLC, based on Ph. Eur. 1742);
(f) Particulate matter (Current USP <788>);
(g) Sterility (Current USP <71> / based Ph. Eur. 2.6.1);
(h) Appearance in use (visual examination);
(i) Spray content by weight (gravimetric mass);
(j) Spray content by assay (Ultra Performance Liquid Chromatography (UPLC) or High Preformanc Liquid Chromatography (HPLC)); and
(k) Weight loss (gravimetric mass).

Testing of these parameters was evaluated according to the schedule listed in Table 14, below.

**Table 14: Parameters Tested and Timing**

| **200µL Vials, Horizontal** | | |
|---|---|---|
| **Time** | **Long Term (25°C/40%RH)** | **Accelerated (40°C/20%RH)** |
| initial | a, b, c, d, e, f, g | not tested |
| 6 mo. | a, b, c, d, e, f | a, b, c, d, e, f, k |
| 12 mo. | a, b, c, d, e, f, g, k | not tested |

| **Device, Horizontal** | | |
|---|---|---|
| **Time** | **Long Term (25°C/40%RH)** | **Accelerated (40°C/20%RH)** |
| initial | h, l, j | not tested |
| 6 mo. | h, l, j | h, l, j |
| 12 mo. | h, l, j | not tested |

### Results:

**Appearance:** No substantial stability related changes were observed during storage at the different storage conditions. **Assay of S-ketamine HCl:** No substantial stability related changes were observed during storage at the different storage conditions. **Chromatographic Purity:** No substantial stability related changes were observed during storage at the different storage conditions. **pH:** No substantial stability related changes were observed during storage at the different storage conditions. **Stereo-isomeric Purity:** No substantial stability related changes were observed during storage at the different storage conditions. **Particulate Matter:** A slight decrease in particulate matter was observed during storgae at the different storage conditions. **Sterility:** No substantial stability related changes were observed during storage after 12 months. **Appearance in Use:** No substantial stability related changes were observed during storage at the different storage conditions. **Spray Content Uniformity by Weight:** No substantial stability related changes were observed during storage at the different storage conditions. **Spray Content Uniformaity by Assay:** No substantial stability related changes were observed during storage at the different storage conditions. **Weight Loss:** Weight loss was observed during storage and was more pronounced at accelerated storage conditions.

### Example 6

### 3 Month Stability Evaluation, S-Ketamine HCl Nasal Formuation

The following S-ketamine HCl formuation was prepared comprising the components listed below.

| **Excipient** | **Concentration** |
|---|---|
| S-Ketamine Hydrochloride = | 161.4 mg/mL |
| Citric acid 1 aqua = | 1.5 mg/mL |
| Disodium edetate = | 0.12 mg/mL |
| Sodium hydroxide all use = | q.s. ad pH 4.5 |
| Water for Injection = | q.s. ad 1 ml |

The stability of the S-ketamine HCl eq. 140 mlg/mL formulation, packed in clear glass vials or in devices was evaluated through 3 months at Initial (Time = 0), Eight Hours, Two Weeks, One Month and Three Months. Three sets of paramaters were evaluated for the formulation stored in glass containers or devices.

### PART A:

The test parameters (and test methods) used in the evaluation were as listed in Table 15, below. The temperature, humidity, illumination, formulation container (glass container / vial or device) and storage position (horizontal or upright) used in the evaluation were listed in Table 16. Where a given set of conditions was tested with more than one sample, the number of samples is listed as n=#.

**Table 15: Testing Parameters and Test Methods (PART A)**

| **Test** | **Test Method(s) Employed** |
|---|---|
| Appearance | Visual Evaluation |
| Assay/Purity and Identification | UPLC |
| pH | USP <791>, based on Ph. Eur. 2.2.3, USP 36.1 , EP 7.8 |
| Chiral Purity | HPLC, based on Ph. Eur. 1742 |
| Spray Content Uniformity by weight and by assay | Gravimetric mass, HPLC or UPLC |
| Microbiological Purity | USP <61>, based on Ph. Eur. 2.6.12, USP 36.1, EP 7.8 |
| Spray Pattern | Laser Diffraction, High Speed Photographic Recording |
| Spray Droplet Size Distribution | Laser Diffraction |
| Color and Clarity | based on Ph.Eur-2.2.1; Ph.Eur-2.2.2, method II, EP 7.8 |

**Table 16: 3 Month Stability Study testing Conditions (PART A)**

| **Initial** | | |
|---|---|---|
| **Glass Cartridge** | @ | Ambient |
| **Device** | @ | Ambient |

| **Eight hours** | | |
|---|---|---|
| **Glass Cartridge** | @ | CIE85-ID65 (light source) 700 W/m², |
| | | Horizontal (n=2) |
| **Device** | @ | CIE85-ID65 (light source) 700 W/m², Horizontal |

| **Two Weeks** | | |
|---|---|---|
| **Glass Cartridge** | @ | 60 °C Horizontal |
| | @ | -20 °C/30 °C 24 hr cycles Horizontal |
| | @ | 5 °C/40 °C 24 hr cycles Horizontal |
| **Device** | @ | -20 °C/30 °C 24 hr cycles Horizontal |
| | @ | 5 °C/40 °C 24 hr cycles Horizontal |

| **One Month** | | |
|---|---|---|
| **Glass Cartridge** | @ | 25 °C/40% RH Horizontal (n=2) |
| | @ | 30 °C/40% RH Horizontal (n=2_ |
| | @ | 30 °C/40% RH Upright (n=2) |
| | @ | 40 °C/20% RH Horizontal (n=3) |
| | @ | 40 °C/20% RH Upright (n=3) |
| | @ | 50 °C Horizontal (n=2) |
| **Device** | @ | 25 °C/40% RH Horizontal |
| | @ | 30 °C/40% RH Horizontal |
| | @ | 30 °C/40% RH Upright |
| | @ | 40 °C/20% RH Horizontal |
| | @ | 40 °C/20% RH Upright |
| | @ | 50 °C Horizontal |

| **Three Months** | | |
|---|---|---|
| **Glass Cartridge** | @ | 5 °C Horizontal (n=2) |
| | @ | 30 °C/40% RH Horizontal (n=3) |
| | @ | 40 °C/20% RH Horizontal (n=3) |
| | @ | 50 °C Horizontal (n=2) |
| **Device** | @ | 5 °C Horizontal (n=2) |
| | @ | 30 °C/40% RH Horizontal |
| | @ | 40 °C/20% RH Horizontal |

### PART B:

The test parameters (and test methods) used in the evaluation were as listed in Table 17, below. The temperature, humidity, illumination, formulation container (glass container / vial or device) and storage position (horizontal or upright) used in the evaluation were listed in Table 18. Where a given set of conditions was tested with more than one sample, the number of samples is listed as n=#.

**Table 17: Testing Parameters and Test Methods (PART B)**

| **Test** | **Test Method(s) Employed** |
|---|---|
| Assay | UPLC |
| Weight Loss | Gravimetric Mass |
| Microbiological Purity | Current USP <61>, based on Ph. Eur. 2.6.12, USP 36.1, EP 7.8 |
| Particulate Matter | Current USP <788>, USP 36.1 |
| Extractable Volume | Current USP <1151 > (with USP<1 >) / based on Ph.Eur.2.9.17, USP 36.1, EP 7.8 |
| Spray Pattern | Laser Diffraction, High Speed Photographic Recording |
| Spray Droplet Size Distribution | Laser Diffraction |
| Actuation Force | Force Profile Recording |
| Plume Geometry | High Speed Photographic Recording |
| Osmolality | USP <785>, based on Ph.Eur.2.2.35, USP 36.1 , EP 7.8 |

**Table 18: 3 Month Stability Study testing Conditions (PART B)**

| **Initial** | | |
|---|---|---|
| **Glass Cartridge** | @ | Ambient (n=2) |
| **Device** | @ | Ambient |
| | @ | 5 °C/Ambient Horizontal |
| | @ | 5 °C/Ambient Vertical Upright |
| | @ | 25 °C/40% RH Horizontal |
| | @ | 30 °C/40% RH Horizontal |
| | @ | 30 °C/40% RH Vertical Upright |
| | @ | 40 °C/20% RH Horizontal |
| | @ | 40 °C/20% RH Vertical Upright |
| | @ | 50 °C Horizontal |
| | @ | 60 °C Horizontal |
| | @ | 25 °C/60% RH Horizontal |
| | @ | Cycling -20 °C/ 30 °C |
| | @ | Cycling +5 °C/40 °C |

| **Eight hours** | | |
|---|---|---|
| **Glass Cartridge** | @ | CIE85-ID65 (light source) 700W/m² at 25 °C/60% RH Horizontal (n=2) |
| **Device** | @ | CIE85-ID65 (light source) 700W/m² |
| | | at 25 °C/60% RH Horizontal |

| **Two Weeks** | | |
|---|---|---|
| **Device** | @ | 60 °C Horizontal |
| | @ | -20 °C/30 °C 24 hr cycles Horizontal |
| | @ | 5 °C/40 °C 24 hr cycles Horizontal |

| **One Month** | | |
|---|---|---|
| **Device** | @ | 25 °C/40% RH Horizontal (n=2) |
| | @ | 30 °C/40% RH Horizontal (n=2) |
| | @ | 30 °C/40% RH Upright (n=2) |
| | @ | 40 °C/20% RH Horizontal (n=2) |
| | @ | 40 °C/20% RH Upright (n=2) |
| | @ | 50 °C Horizontal (n=2) |

| **Three Months** | | |
|---|---|---|
| **Device** | @ | 5 °C Horizontal |
| | @ | 30 °C/40% RH Horizontal |
| | @ | 40 °C/20% RH Horizontal (n=2 |
| | @ | 50 °C Horizontal (n=2) |

### PART C:

The test parameters (and test methods) used in the evaluation were as listed in Table 19, below. The temperature, humidity, illumination, formulation container (glass container / vial or device) and storage position (horizontal or upright) used in the evaluation were listed in Table 20. Where a given set of conditions was tested with more than one sample, the number of samples is listed as n=#.

**Table 19: Testing Parameters and Test Methods (PART C)**

| **Test** | **Test Method(s) Employed** |
|---|---|
| Spray Content Uniformity by weight and by assay | Gravimetric mass and HPLC or UPLC |
| Spray Pattern | Laser Diffraction, High Speed Photographic Recording |
| Spray Droplet Size Distribution | Laser Diffraction |
| Actuation Force | Force profile recording |
| Plume Geometry | High Speed Photographic Recording |
| Assay of NaEDTA | HPLC |

**Table 20: 3 Month Stability Study testing Conditions (PART C)**

| **Initial** | | |
|---|---|---|
| **Glass Cartridge** | @ | Ambient |
| **Device** | @ | Ambient |

| **Eight hours** | | |
|---|---|---|
| **Device** | @ | CIE85-ID65 (light source) 700 W/m² at 25 °C/60% RH |

| **Two Weeks** | | |
|---|---|---|
| **Glass Cartridge** | @ | 60 °C Horizontal |
| **Device** | @ | 60 °C Horizontal |
| | @ | -20 °C/30 °C 24 hr cycles Horizontal (n=2) |

| **One Month** | | |
|---|---|---|
| **Glass Cartridge** | @ | 40 °C/20% RH Horizontal |
| | @ | 40 °C/20% RH Upright |
| **Device** | @ | 25 °C/40% RH Horizontal |
| | @ | 30 °C/40% RH Horizontal |
| | @ | 30 °C/40% RH Upright |
| | @ | 40 °C/20% RH Horizontal |
| | @ | 40 °C/20% RH Upright |
| | @ | 50 °C Horizontal |

| **Three Months** | | |
|---|---|---|
| **Glass Cartridge** | @ | 30 °C/40% RH Horizontal |
| | @ | 40 °C/20% RH Horizontal |
| **Device** | @ | 5 °C Horizontal |
| | @ | 30 °C/40% RH Horizontal |
| | @ | 40 °C/20% RH Horizontal |
| | @ | 50 °C Horizontal |

### Results:

**Initial Interval:** Initial testing was performed in vials and devices. No deviations occurred and no investigations were performed for the initial analysis. **Eight Hour Interval:** Eight hour testing was performed in vials and devices. No deviations occurred for the eight hour analysis. **Two Week Interval:** Two week testing was performed in vials and devices. No deviations occurred for the two week analysis. **One Month Interval:** One month testing was performed in vials and devices. No deviations occurred for the two week analysis. **Three Month Interval:** One month testing was performed in vials and devices. No deviations occurred for the two week analysis.

## Claims

1. A pharmaceutical composition comprising S-ketamine hydrochloride and water; wherein the pharmaceutical composition does not contain an antimicrobial preservative;
wherein the S-ketamine hydrochloride is present in a concentration of at least eq. 120 mg/mL, based on the total volume of the composition.

2. The pharmaceutical composition according to Claim 1, which
(i) additionally contains a buffer; and/or
(ii) has a pH value within the range of from 4.0 to 6.5.

3. The pharmaceutical composition according to Claim 1, wherein the S-ketamine hydrochloride is present in a concentration in the range of eq. 125 mg/mL to eq. 150 mg/mL, based on the total volume of the composition; or wherein the S-ketamine hydrochloride is present in a concentration in the range of 126 mg / mL to 162 mg/mL, based on the total volume of the composition.

4. The pharmaceutical composition according to Claim 1, wherein the pharmaceutical composition is formulated for nasal administration.

5. The pharmaceutical composition according to Claim 1, wherein the pharmaceutical composition further contains a buffer.

6. The pharmaceutical composition according to Claim 5, wherein the buffer is 1N NaOH.

7. The pharmaceutical composition according to Claim 5, wherein the buffer is present in an amount sufficient to adjust the pH of the pharmaceutical composition to a pH in the range of from pH 3.5 to pH 6.5; or
wherein the buffer is present in an amount sufficient to adjust the pH of the composition to a pH in the range of from pH 4.5 to pH 5.5.

8. The pharmaceutical composition according to Claim 1, wherein the pharmaceutical composition exhibits a shelf-life under accelerated storage conditions of at least 3 months.

9. A pharmaceutical dosage form comprising the pharmaceutical composition according to any of claims 1-8.

10. The dosage form according to claim 9, which is adapted for nasal administration.

11. The pharmaceutical composition according to Claim 1 or the pharmaceutical dosage form according to Claim 9 for use in the treatment of depression.

12. The pharmaceutical composition or the pharmaceutical dosage form for use according to claim 11, wherein the depression is selected from the group consisting of major depressive disorder, unipolar depression, treatment-refractory depression, resistant depression, anxious depression and bipolar depression.

13. The pharmaceutical composition or the pharmaceutical dosage form for use according to claim 11, wherein the depression is selected from the group consisting of resistant depression or treatment refractory depression.

14. A pharmaceutical composition as in Claim 1, comprising S-ketamine HCl; citric acid; disodium edetate; sodium hydroxide; and water.

15. A pharmaceutical composition as in Claim 1, comprising
(a) S-ketamine HCl; wherein the S-Ketamine HCl is present in an amount of about 161.4 mg/ml;
(b) citric acid 1 aqua; wherein the citric acid is present in an amount of about 1.5 mg/ml;
(c) disodium edetate; wherein the disodium edetate is present in an amount of about 0.12 mg/ml;
(d) sodium hydroxide; wherein the sodium hydroxide is present in an amount sufficient to adjust the pH of the pharmaceutical composition to a pH of about 4.5; and
(e) water.

## Patentansprüche

1. Pharmazeutische Zusammensetzung, umfassend S-Ketaminhydrochlorid und Wasser, wobei die pharmazeutische Zusammensetzung keinen antimikrobiellen Konservierungsstoff enthält,
wobei das S-Ketaminhydrochlorid in einer Konzentration von mindestens Äq. 120 mg/ml, bezogen auf das Gesamtvolumen der Zusammensetzung, vorliegt.

2. Pharmazeutische Zusammensetzung nach Anspruch 1, die
(i) zusätzlich einen Puffer enthält und/oder
(ii) einen pH-Wert im Bereich von 4,0 bis 6,5 aufweist.

3. Pharmazeutische Zusammensetzung nach Anspruch 1, wobei das S-Ketaminhydrochlorid in einer Konzentration im Bereich von äq. 125 mg/ml bis äq. 150 mg/ml, bezogen auf das Gesamtvolumen der Zusammensetzung, vorliegt oder wobei das S-Ketaminhydrochlorid in einer Konzentration im Bereich von 126 mg/ml bis 162 mg/ml, bezogen auf das Gesamtvolumen der Zusammensetzung, vorliegt.

4. Pharmazeutische Zusammensetzung nach Anspruch 1, wobei die pharmazeutische Zusammensetzung für die nasale Verabreichung formuliert ist.

5. Pharmazeutische Zusammensetzung nach Anspruch 1, wobei die pharmazeutische Zusammensetzung weiterhin einen Puffer enthält.

6. Pharmazeutische Zusammensetzung nach Anspruch 5, wobei es sich bei dem Puffer um 1 N NaOH handelt.

7. Pharmazeutische Zusammensetzung nach Anspruch 5, wobei der Puffer in einer Menge vorliegt, die ausreicht, um den pH-Wert der pharmazeutischen Zusammensetzung auf einen pH im Bereich von pH 3,5 bis pH 6,5 einzustellen, oder
wobei der Puffer in einer Menge vorliegt, die ausreicht, um den pH-Wert der Zusammensetzung auf einen pH im Bereich von pH 4,5 bis pH 5,5 einzustellen.

8. Pharmazeutische Zusammensetzung nach Anspruch 1, wobei die pharmazeutische Zusammensetzung unter beschleunigten Lagerungsbedingungen eine Haltbarkeit von mindestens 3 Monaten aufweist.

9. Pharmazeutische Dosierungsform, umfassend die pharmazeutische Zusammensetzung nach einem der Ansprüche 1-8.

10. Dosierungsform nach Anspruch 9, die für die nasale Verabreichung ausgelegt ist.

11. Pharmazeutische Zusammensetzung nach Anspruch 1 oder pharmazeutische Dosierungsform nach Anspruch 9 zur Verwendung bei der Behandlung von Depression.

12. Pharmazeutische Zusammensetzung oder pharmazeutische Dosierungsform zur Verwendung nach Anspruch 11, wobei die Depression aus der aus schwerer Depression, unipolarer Depression, behandlungsrefraktärer Depression, resistenter Depression, Angstdepression und bipolarer Depression bestehenden Gruppe ausgewählt ist.

13. Pharmazeutische Zusammensetzung oder pharmazeutische Dosierungsform zur Verwendung nach Anspruch 11, wobei die Depression aus der aus resistenter Depression und behandlungsrefraktärer Depression bestehenden Gruppe ausgewählt ist.

14. Pharmazeutische Zusammensetzung nach Anspruch 1, umfassend S-Ketamin-HCl, Citronensäure, Dinatriumedetat, Natriumhydroxid und Wasser.

15. Pharmazeutische Zusammensetzung nach Anspruch 1, umfassend
(a) S-Ketamin-HCl, wobei das S-Ketamin-HCl in einer Menge von etwa 161,4 mg/ml vorliegt,
(b) Citronensäure x H₂O, wobei die Citronensäure in einer Menge von etwa 1,5 mg/ml vorliegt,
(c) Dinatriumedetat, wobei das Dinatriumedetat in einer Menge von etwa 0,12 mg/ml vorliegt,
(d) Natriumhydroxid, wobei das Natriumhydroxid in einer Menge vorliegt, die ausreicht, um den pH-Wert der pharmazeutischen Zusammensetzung auf einen pH von etwa 4,5 einzustellen, und
(e) Wasser.

## Revendications

1. Composition pharmaceutique comprenant du chlorhydrate de S-kétamine et de l'eau ; la composition pharmaceutique ne contenant pas de conservateur antimicrobien ;
dans laquelle le chlorhydrate de S-kétamine est présent en une concentration d'au moins 120 mg d'éq./ml, par rapport au volume total de la composition.

2. Composition pharmaceutique selon la revendication 1, qui
(i) contient de plus un tampon ; et/ou
(ii) a une valeur de pH dans la plage de 4,0 à 6,5.

3. Composition pharmaceutique selon la revendication 1, dans laquelle le chlorhydrate de S-kétamine est présent en une concentration dans la plage de 125 mg d'éq./ml à 150 mg d'éq./ml, par rapport au volume total de la composition ; ou dans laquelle le chlorhydrate de S-kétamine est présent en une concentration dans la plage de 126 mg/ml à 162 mg/ml, par rapport au volume total de la composition.

4. Composition pharmaceutique selon la revendication 1, la composition pharmaceutique étant formulée pour l'administration nasale.

5. Composition pharmaceutique selon la revendication 1, la composition pharmaceutique contenant en outre un tampon.

6. Composition pharmaceutique selon la revendication 5, dans laquelle le tampon est du NaOH 1N.

7. Composition pharmaceutique selon la revendication 5, le tampon étant présent en une quantité suffisante pour ajuster le pH de la composition pharmaceutique à un pH dans la plage de pH 3,5 à pH 6,5 ; ou
le tampon étant présent en une quantité suffisante pour ajuster le pH de la composition à un pH dans la plage de pH 4,5 à pH 5,5.

8. Composition pharmaceutique selon la revendication 1, la composition pharmaceutique présentant une durée limite de stockage dans des conditions de stockage accélérées d'au moins 3 mois.

9. Forme galénique pharmaceutique comprenant la composition pharmaceutique selon l'une quelconque des revendications 1-8.

10. Forme galénique selon la revendication 9, qui est adaptée à l'administration nasale.

11. Composition pharmaceutique selon la revendication 1 ou forme galénique pharmaceutique selon la revendication 9 destinées à être utilisées dans le traitement d'une dépression.

12. Composition pharmaceutique ou forme galénique pharmaceutique destinées à être utilisées selon la revendication 11, la dépression étant choisie dans le groupe constitué par un trouble dépressif majeur, une dépression unipolaire, une dépression réfractaire aux traitements, une dépression résistante, une dépression anxieuse et une dépression bipolaire.

13. Composition pharmaceutique ou forme galénique pharmaceutique destinées à être utilisées selon la revendication 11, la dépression étant choisie dans le groupe constitué par une dépression résistante ou une dépression réfractaire aux traitements.

14. Composition pharmaceutique selon la revendication 1, comprenant de la S-kétamine HGl ; de l'acide citrique ; de l'édétate disodique ; de l'hydroxyde de sodium ; et de l'eau.

15. Composition pharmaceutique selon la revendication 1, comprenant
(a) de la S-kétamine HGl ; la S-kétamine HCl étant présente en une quantité d'environ 161,4 mg/ml ;
(b) de l'acide citrique monohydraté ; l'acide citrique étant présent en une quantité d'environ 1,5 mg/ml ;
(c) de l'édétate disodique ; l'édétate disodique étant présent en une quantité d'environ 0,12 mg/ml ;
(d) de l'hydroxyde de sodium ; l'hydroxyde de sodium étant présent en une quantité suffisante pour ajuster le pH de la composition pharmaceutique à un pH d'environ 4,5 ; et
(e) de l'eau.
